# EUROPÄISCHE PATENTSCHRIFT

(11) **EP 3 122 328 B1**
(45) Veröffentlichungstag und Bekanntmachung des Hinweises auf die Patenterteilung: **01.05.2019**
(21) Anmeldenummer: 15707129.1
(22) Anmeldetag: 27.02.2015
(51) Int. Cl.: A61K 8/58, A61Q 5/02, A61Q 5/12

(54) **SILAN ENTHALTEND JEWEILS MINDESTENS ZWEI ALKOXYGRUPPEN SOWIE EINE GUANIDINO- ODER HARNSTOFFGRUPPE**
SILANE CONTAINING RESPECTIVELY AT LEAST TWO ALKOXY GROUPS AND A GUANIDONO OR CARBAMIDE GROUP
SILANE CONTENANT RESPECTIVEMENT AU MOINS DEUX GROUPES ALKOXY ET UN GROUPE GUANIDINO OU CARBAMATE

(30) Priorität: 28.03.2014 DE 102014205806
(43) Veröffentlichungstag der Anmeldung: 01.02.2017
(73) Patentinhaber: Evonik Degussa GmbH, 45128 Essen (DE)
(72) Erfinder: TRAMBITAS, Alexandra, 45130 Essen (DE); FIEDEL, Olga, 45131 Essen (DE); HARTUNG, Christian, 45133 Essen (DE)
(74) Vertreter: Evonik Patent Association
(86) Internationale Anmeldenummer: PCT/EP2015/054145
(87) Internationale Veröffentlichungsnummer: WO 2015/144384

(56) Entgegenhaltungen:
- EP-A2- 0 159 628
- EP-A2- 1 862 198
- WO-A1-2004/012691
- WO-A2-2012/076293
- WO-A2-2013/068966
- DE-A1- 2 704 905
- DE-A1-102005 004 704
- US-A1- 2008 008 882
- US-A1- 2011 046 299

## Beschreibung

### Gebiet der Erfindung

Gegenstand der Erfindung sind Haarpflegeformulierungen enthaltend Silane, welche jeweils mindestens zwei Alkoxygruppen sowie eine Guanidinogruppe aufweisen, sowie verschiedenste Verwendungen der vorgenannten Silane zur Haarpflege und zum Haarschutz.

### Stand der Technik

Die EP0159628 offenbart Alkyltrialkoxysilane und deren Verwendung zur Stärkung der Zugfestigkeit von Haaren.

WO2004012691 offenbart die Verwendung von Aminogruppen enthaltenden Trimethoxysilanen in einem kosmetischen Mittel zur Verbesserung des Zustandes von Haaren.

WO2012076293 offenbart die Verwendung von polyaminofunktionellen Polysiloxanen in Formulierungen sowie die Formulierungen, die diese Polysiloxane enthalten.

Die DE102005004704 offenbart bestimmte guanidinogruppen-haltige Siloxane und deren Verwendung für kosmetische Formulierungen.

### Beschreibung der Erfindung

Überraschenderweise wurde gefunden, dass die beschriebenen Silane hervorragende Haarbehandlungsmittel darstellen und die bisher im Stand der Technik beschriebenen in ihren Anwendungsergebnissen übertreffen.

Gegenstand der vorliegenden Erfindung ist daher die Verwendung von Silanen bzw. Haarpflegeformulierungen enthaltend diese Silane, welche mindestens zwei Alkoxygruppen sowie eine Guanidino gruppe aufweisen wie in Anspruch 1 beschrieben.

Ein Vorteil der offenbarten Formulierungen ist, dass diese hervorragend enthaltene Wirkstoffe auf Haar zu depositionieren vermögen.

Noch ein Vorteil der offenbarten Formulierungen ist, dass diese das Haar griffiger machen und zur Haarfestigung beitragen.
Noch ein Vorteil der offenbarten Formulierungen ist, dass diese dem Haar Glanz verleihen.
Noch ein Vorteil der offenbarten Formulierungen ist, dass diese das Haar länger anhaltend (über mehrere Shampoobehandlungen hinweg) konditionieren.
Noch ein Vorteil der offenbarten Formulierungen ist, dass diese die Kopfhaut weich und geschmeidig anfühlen lassen.
Noch ein Vorteil der offenbarten Formulierungen ist, dass diese das Haar schneller trocknen lassen.
Noch ein Vorteil der offenbarten Formulierungen ist, dass diese die Waschbeständigkeit der Farbe von gefärbten Haaren verbessern.
Noch ein Vorteil der offenbarten Formulierungen ist, dass diese dem Haar eine erhöhte Hitzebeständigkeit verleihen.
Noch ein Vorteil der offenbarten Formulierungen ist, dass diese eine gute Lagerstabilität bzgl. Viskosität und Aussehen (wie Klarheit oder Einphasigkeit) aufweisen und das auch bei erhöhten Temperaturen von 40 °C.
Noch ein Vorteil der offenbarten Formulierungen ist, dass diese im Falle von Färbeformulierungen zu einer verbesserten Einfärbbarkeit von Haaren führt.
Noch ein Vorteil der offenbarten Formulierungen ist, dass diese das Haar staub- und schmutzabweisend machen.
Noch ein Vorteil der offenbarten Formulierungen ist, dass diese das Haar weniger bruchanfällig machen.
Noch ein Vorteil der offenbarten Formulierungen ist, dass sich diese leicht herstellen lassen.

Beansprucht wird somit eine Verwendung eines Silans der allgemeinen Formel 1 oder einer Haarpflegeformulierung enthaltend
mindestens ein Silan der allgemeinen Formel 1 enthaltend
zwei oder drei Alkoxygruppen,
einen Rest enthaltend mindestens eine Guanidino
gruppe,
mit der Maßgabe, dass eine Alkylgruppe enthalten ist, wenn das Silan zwei Alkoxygruppen enthält,
dadurch gekennzeichnet, dass das Silan ausgewählt ist aus solchen der
allgemeinen Formel 1

R¹ₙXSi(OR²)₃₋ₙ (Formel 1)

mit
n= 0 oder 1, insbesondere 1
R¹= CH₃,
R² = CH₃ oder CH₂CH₃, insbesondere CH₂CH3,
X= R³Y,
R³ = lineare oder verzweigte, gesättigte oder ungesättigte, divalente Kohlenwasserstoffgruppe mit 1 bis 20, bevorzugt 1 bis 6 Kohlenstoffatomen und insbesondere eine -(CH₂)₃-Gruppe,
Y = organischer Rest umfassend mindestens eine Guanidinogruppe und zusätzlich mindestens eine weitere Aminogruppe, insbesondere eine primäre oder sekundäre Aminogruppe
zur Erhöhung der Grifffestigkeit und/oder
zur Verbesserung der Nasskämmbarkeit und/oder
zur Erhöhung des Glanzes von Haaren.

Alle angegebenen Prozent (%) sind, wenn nicht anders angegeben, Massenprozent.

Erfindungsgemäß bevorzugt verwendete Haarpflegeformulierungen sind dadurch gekennzeichnet, dass das Silan genau eine Alkylgruppe und genau zwei Alkoxygruppen enthält.

Erfindungsgemäß bevorzugt verwendete Haarpflegeformulierungen sind dadurch gekennzeichnet, dass die Alkylgruppe im Silan ausgewählt ist aus Methyl-, Ethyl-, Propyl-, Isopropyl-, Butyl-, Hexyl-, Octyl-, wobei Methyl- besonders bevorzugt ist.

Die erfindungsgemäß im Silan enthaltenen Alkoxygruppen können gleich oder verschieden sein, erfindungsgemäß bevorzugt sind sie gleich.

Erfindungsgemäß bevorzugt verwendete Haarpflegeformulierungen sind dadurch gekennzeichnet, dass die Alkoxygruppen im Silan ausgewählt sind aus Methoxy-, Ethoxy-, Propoxy-, Isopropoxy-, Butoxy-, wobei Ethoxy- besonders bevorzugt ist.

Der Begriff "Harnstoffgruppe", wie im Zusammenhang mit der vorliegenden Erfindung verwendet, bedeutet -NRC(O)NR- oder -NRC(O)NR2-Gruppen, wobei R gleich Wasserstoff oder Alkyl oder Aryl bedeuten kann, wobei Wasserstoff bevorzugt ist.
Der Begriff "Guanidinogruppe", wie im Zusammenhang mit der vorliegenden Erfindung verwendet, bedeutet -NHC(NH)NHR-Gruppen, die gegebenenfalls protoniert vorliegen, und wobei R gleich Wasserstoff oder Alkyl oder Aryl bedeuten kann, wobei Wasserstoff bevorzugt ist.

Erfindungsgemäß bevorzugt verwendete Haarpflegeformulierungen sind dadurch gekennzeichnet, dass das Silan in dem Rest enthaltend mindestens eine Guanidino-gruppe zusätzlich eine Aminogruppe enthält.
Der Begriff "Aminogruppe" im Zusammenhang mit der vorliegenden Erfindung umfasst gegebenenfalls protonierte primäre, sekundäre und tertiäre Aminogruppen sowie quartäre Ammoniumgruppen.
Erfindungsgemäß bevorzugt verwendete Haarpflegeformulierungen sind dadurch gekennzeichnet, dass die Aminogruppe ausgewählt ist aus
gegebenenfalls protonierten primären, sekundären und tertiären Aminogruppen, wobei gegebenenfalls protonierte primäre Aminogruppen bevorzugt sind.

Erfindungsgemäß bevorzugt verwendete Haarpflegeformulierungen sind dadurch gekennzeichnet, dass
die Alkoxygruppen im Silan ausgewählt sind aus Ethoxy-,
die Alkylgruppe im Silan ausgewählt ist aus Methyl-,
wobei bevorzugt genau eine Alkylgruppe und genau zwei Alkoxygruppen im Silan enthalten sind.

Erfindungsgemäß bevorzugt verwendete Haarpflegeformulierungen sind dadurch gekennzeichnet, dass
die Alkoxygruppen im Silan ausgewählt sind aus Ethoxy-,
die Alkylgruppe im Silan ausgewählt ist aus Methyl-,
wobei bevorzugt genau eine Alkylgruppe und genau zwei Alkoxygruppen im Silan enthalten sind und
dass der mindestens eine Guanidinogruppe = umfassende Rest optional zusätzlich mindestens eine weitere Aminogruppe enthaltend, insbesondere eine primäre oder sekundäre Aminogruppe, ausgewählt ist aus der Gruppe der Formeln a-g 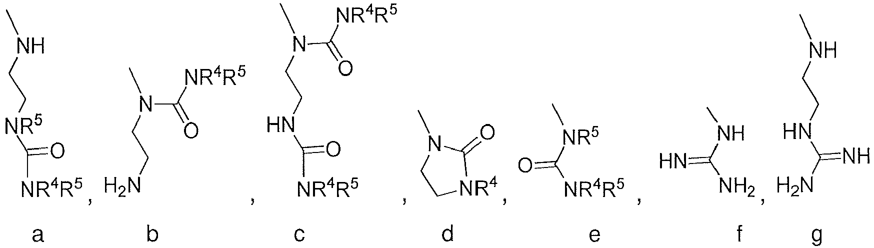 mit R⁴, R⁵ = unabhängig voneinander gleich oder verschieden H, CH₃, Bu oder Ph.

Erfindungsgemäß bevorzugt verwendete Haarpflegeformulierungen sind dadurch gekennzeichnet, dass das Silan ausgewählt ist aus solchen der allgemeinen Formel 1

R¹ₙXSi(OR²)₃₋ₙ (Formel 1)

mit
n= 0 oder 1, insbesondere 1
R¹= CH₃,
R² = CH₃ oder CH₂CH₃, insbesondere CH₂CH₃,
X= R³Y,
R³ = lineare oder verzweigte, gesättigte oder ungesättigte, divalente Kohlenwasserstoffgruppe mit 1 bis 20, bevorzugt 1 bis 6 Kohlenstoffatomen und insbesondere eine -(CH₂)₃-Gruppe,
Y = organischer Rest umfassend mindestens eine Guanidinogruppe und optional zusätzlich mindestens eine weitere Aminogruppe, insbesondere eine primäre oder sekundäre Aminogruppe, bevorzugt ausgewählt aus der Gruppe der Substituenten der Formeln a-g 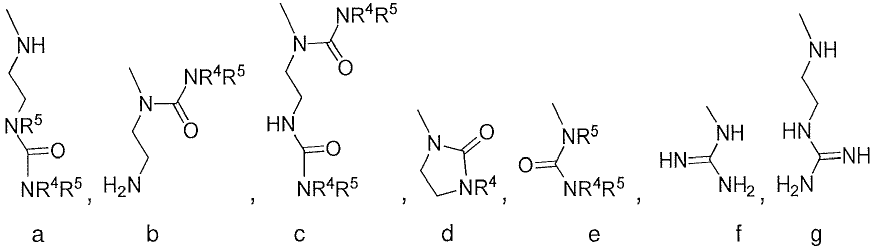
mit R⁴, R⁵ = unabhängig voneinander gleich oder verschieden H, CH₃, Bu oder Ph.

Erfindungsgemäß besonders bevorzugt verwendete Haarpflegeformulierungen sind dadurch gekennzeichnet, dass neben den Silanen zusätzlich mindestens ein Disilan der allgemeinen Formel 2

R¹(OR²)₂SiR³ZR³Si(OR²)₂R¹ (Formel 2),

wobei
Z= zweibindiger organischer Rest umfassend mindestens eine Guanidinogruppe und optional zusätzlich mindestens eine weitere Aminogruppe, insbesondere eine primäre oder sekundäre Aminogruppe, bevorzugt ausgewählt aus der Gruppe der Reste der Formeln h-k 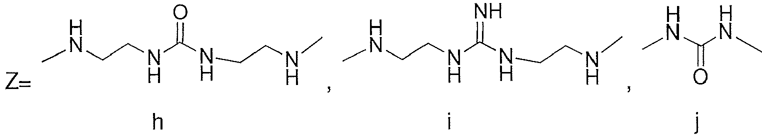 und 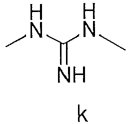 und R¹, R² und R³ wie oben für die allgemeine Formel 1 definiert,
enthalten ist.

Erfindungsgemäß besonders bevorzugt verwendete Haarpflegeformulierungen sind dadurch gekennzeichnet, dass die Silane ausgewählt sind aus der Gruppe:

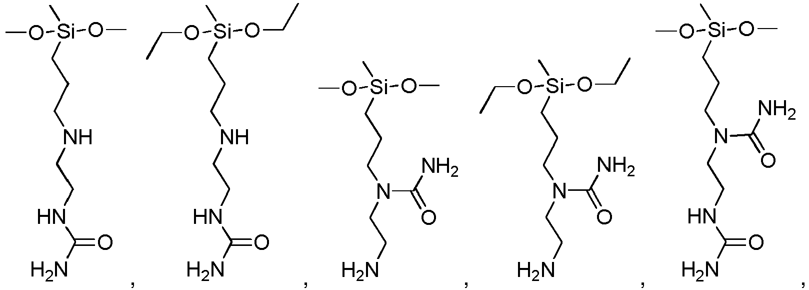

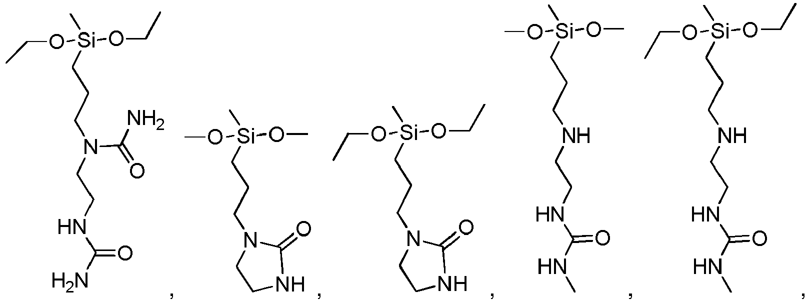

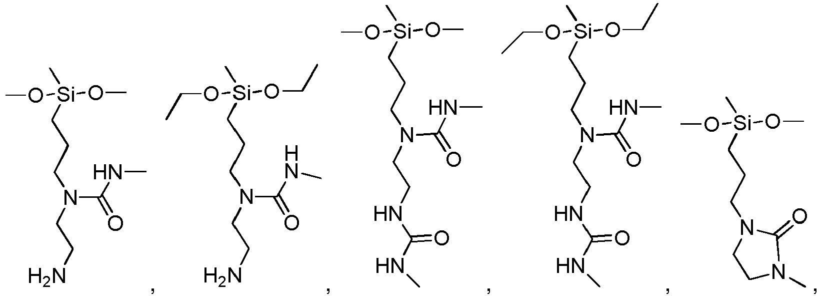

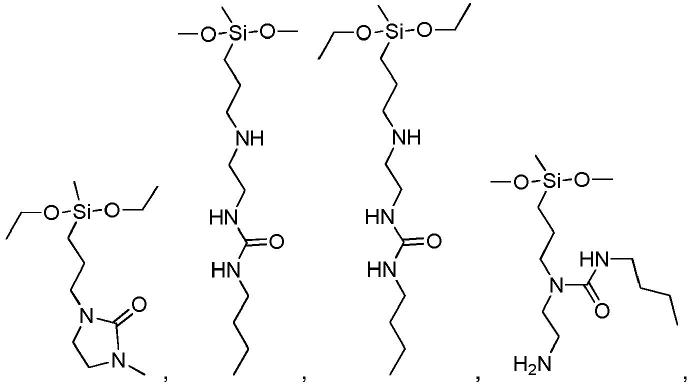

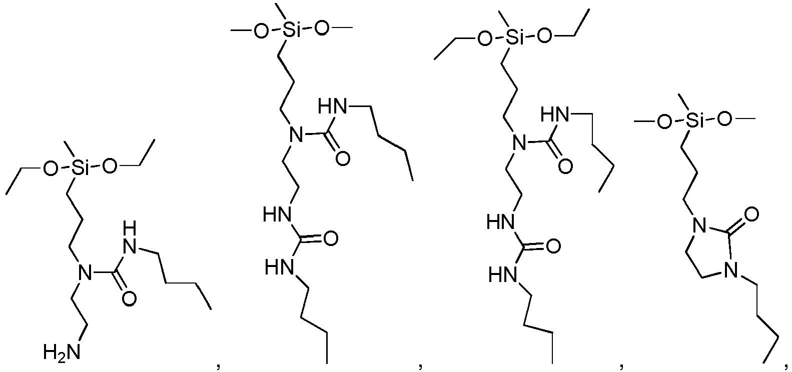

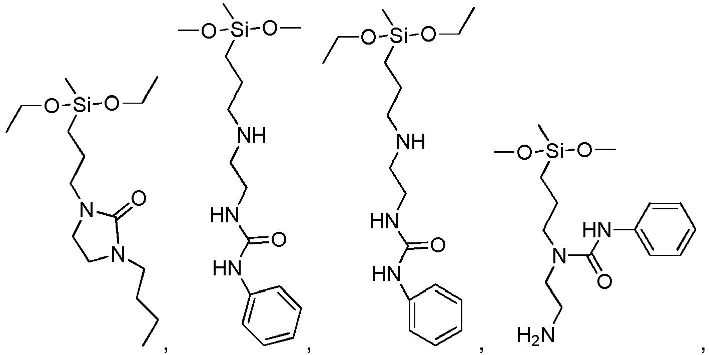

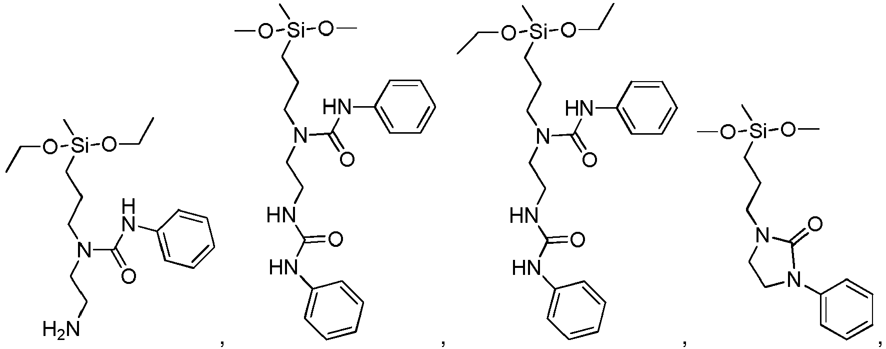

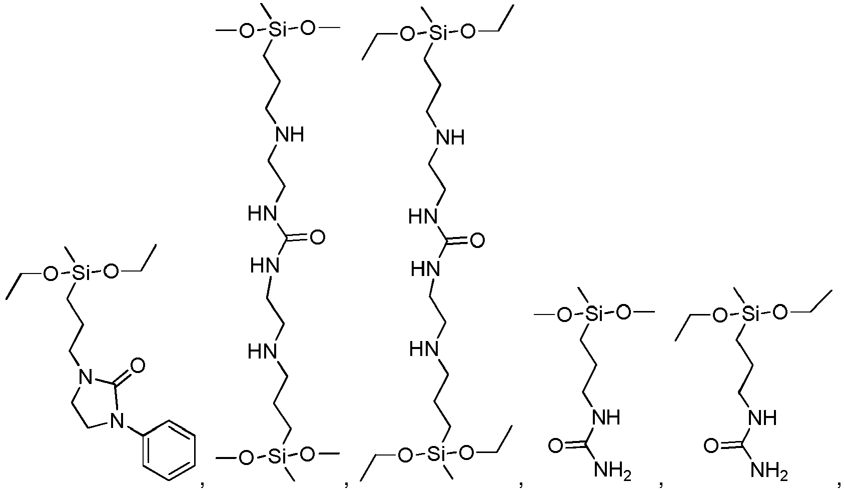

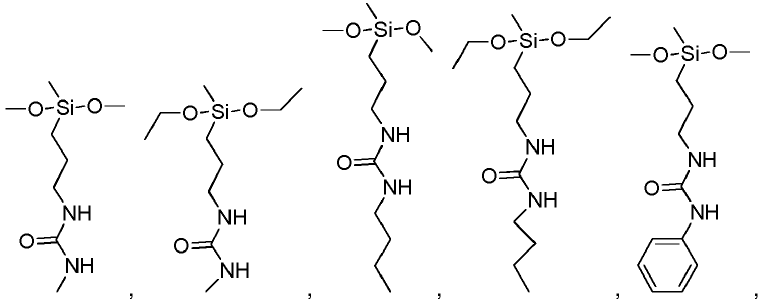

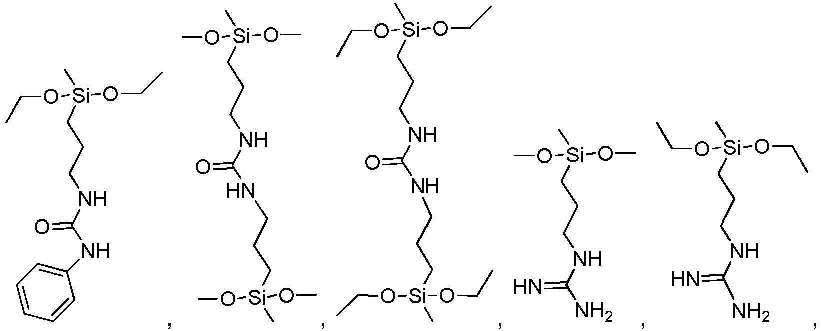

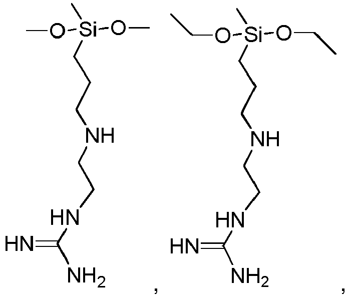

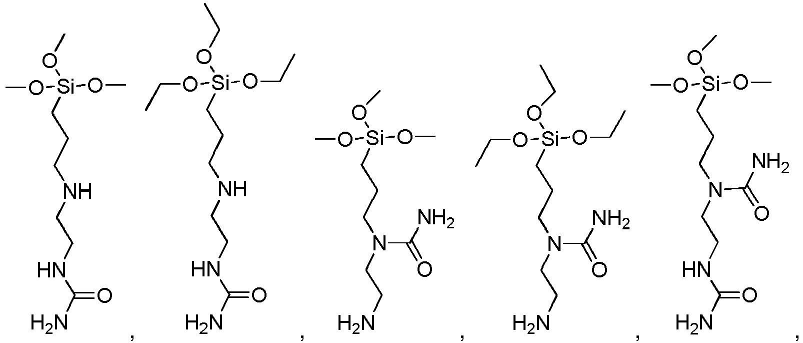

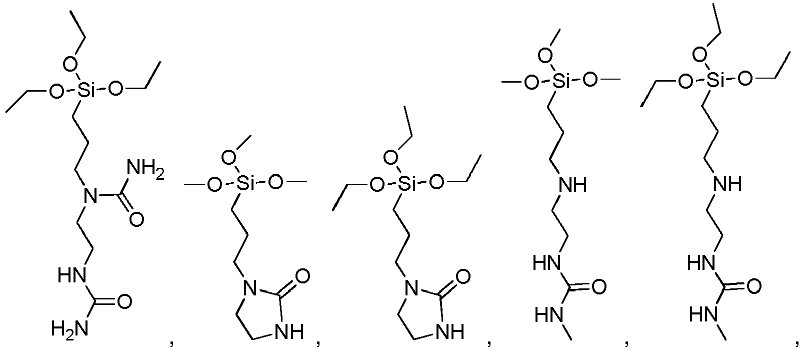

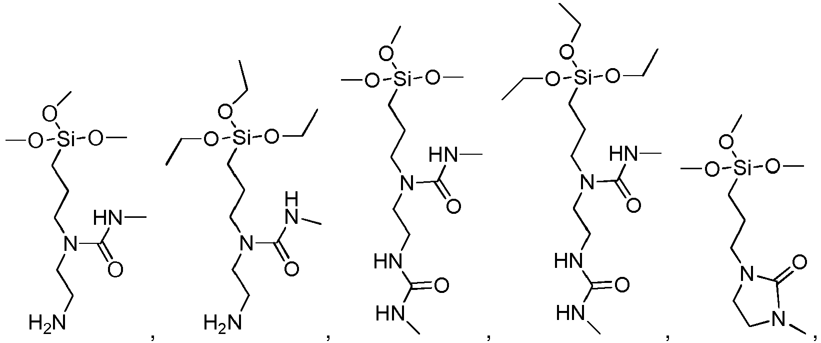

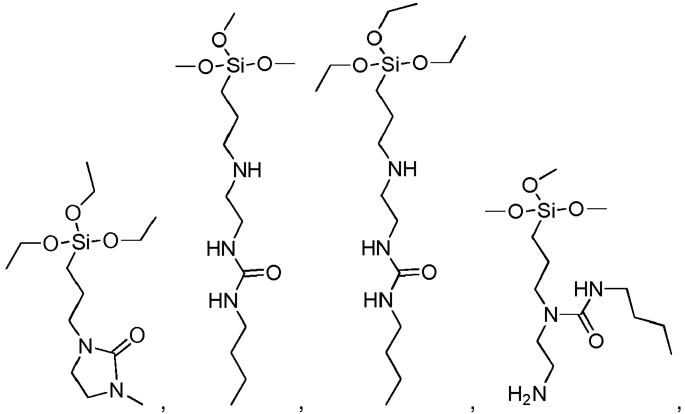

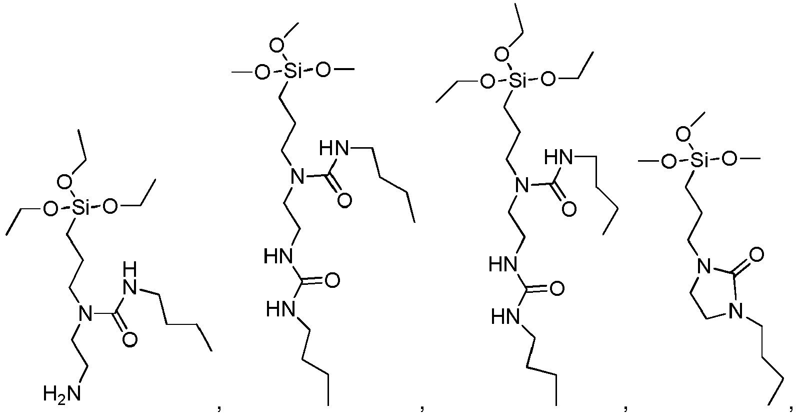

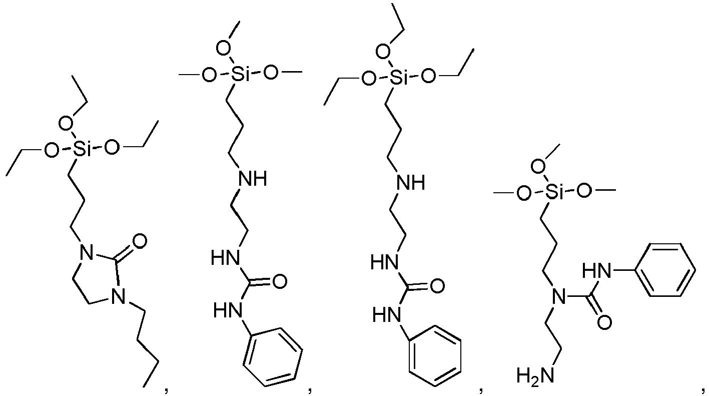

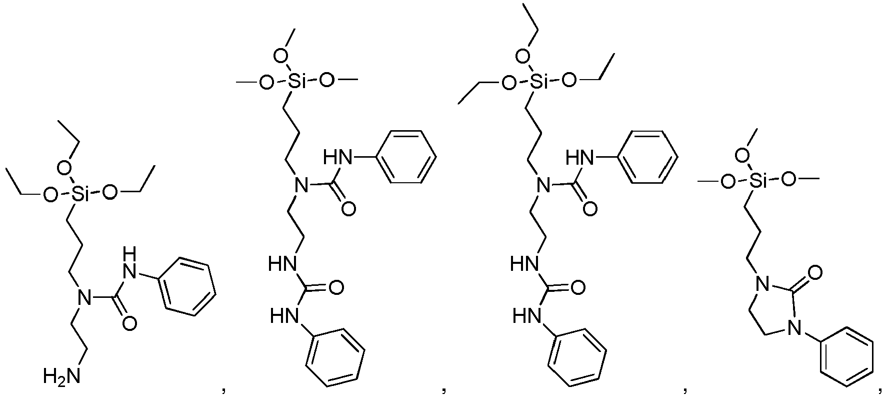

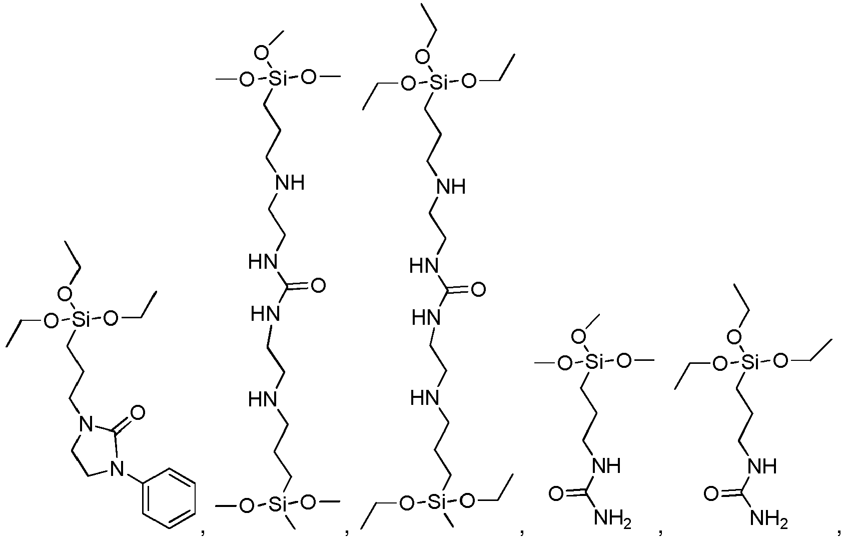

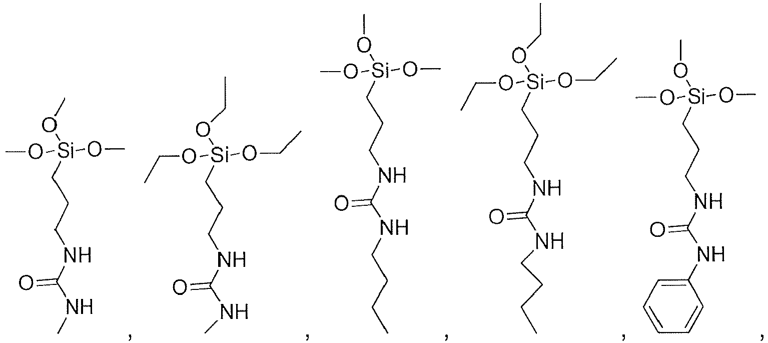

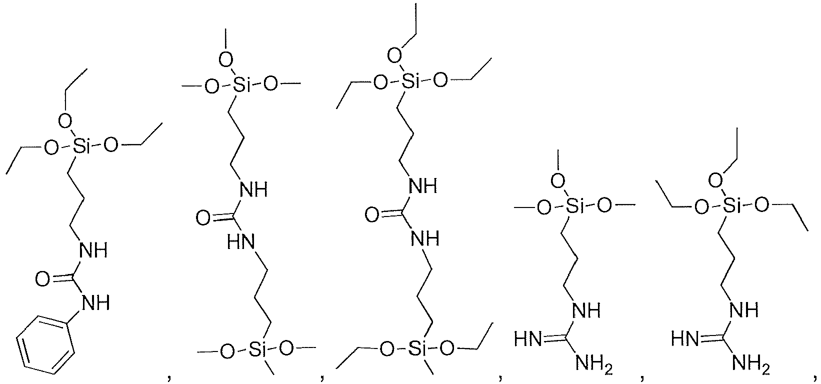

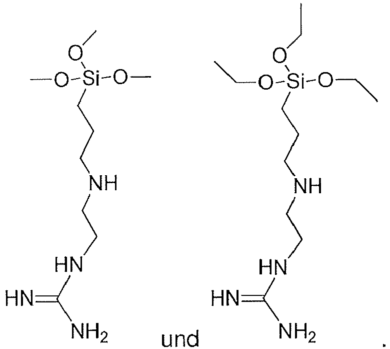

Das mindestens eine Silan ist bevorzugt in der bevorzugt verwendeten Formulierung in einer Menge von 0,1 Gew.-% bis 20 Gew.-%, bevorzugt von 0,3 Gew.-% bis 10 Gew.-%, besonders bevorzugt von 0,5 Gew.-% bis 5 Gew.-%, enthalten, wobei sich die Gewichtsprozente auf die Gesamtformulierung beziehen.

Erfindungsgemäß verwendete Formulierungen, können zum Beispiel mindestens eine zusätzliche Komponente enthalten, ausgewählt aus der Gruppe der
Tenside,
Emollients,
Emulgatoren,
Verdicker/Viskositätsregler/Stabilisatoren,
Antioxidantien,
Hydrotrope (oder Polyole),
Fest- und Füllstoffe,
Perlglanzadditive,
Deodorant- und Antitranspirantwirkstoffe,
Insektrepellentien,
Selbstbräuner,
Konservierungsstoffe,
Konditioniermittel,
Parfüme,
Farbstoffe,
kosmetische Wirkstoffe,
Pflegeadditive,
Überfettungsmittel,
Lösungsmittel.
Substanzen, die als beispielhafte Vertreter der einzelnen Gruppen eingesetzt werden können, sind dem Fachmann bekannt und können beispielsweise der EP2273966A1 entnommen werden. Diese Patentanmeldung wird hiermit als Referenz eingeführt und gilt somit als Teil der Offenbarung.
Bezüglich weiterer fakultativer Komponenten sowie der eingesetzten Mengen dieser Komponenten wird ausdrücklich auf die dem Fachmann bekannten einschlägigen Handbücher, zum Beispiel K. Schrader, "Grundlagen und Rezepturen der Kosmetika", 2. Auflage, Seite 329 bis 341, Hüthig Buch Verlag Heidelberg, verwiesen.
Die Mengen der jeweiligen Zusätze richten sich nach der beabsichtigten Verwendung. Typische Rahmenrezepturen für die jeweiligen Anwendungen sind bekannter Stand der Technik und sind beispielsweise in den Broschüren der Hersteller der jeweiligen Grund- und Wirkstoffe enthalten. Diese bestehenden Formulierungen können in der Regel unverändert übernommen werden. Im Bedarfsfall können zur Anpassung und Optimierung die gewünschten Modifizierungen aber durch einfache Versuche komplikationslos vorgenommen werden.

Bei den erfindungsgemäßen Verwendungen werden Silane entsprechend Ihrer jeweiligen Bevorzugung, mit der diese in den erfindungsgemäß verwendetenen Formulierungen enthalten sind, bevorzugt verwendet.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

### Beispiele

### Beispiel 1: Herstellung einer ethanolischen Lösung enthaltend 3-Guanidinopropylmethyldiethoxysilan

In einem 500-ml-Vierhalskolben mit angeschlossenen KPG-Rührer, Tropftrichter, Rückflusskühler und Innenthermometer wurden 191,34 g 3-Aminopropylmethyldiethoxysilan (Dynasylan 1505, Evonik Industries AG) und 140 g Ethanol vorgelegt. Unter Rühren wurden bei Raumtemperatur 54 g Essigsäure (99-100 %ig, J. T. Baker) in 15 Minuten zugetropft. Es wurde auf 80 °C erhitzt und unter Rühren wurden 21 g Cyanamid F1000 (Alzchem Trostberg GmbH) gelöst in 60 g Ethanol über einen Zeitraum von 2 Stunden zugetropft. Es wurde weitere 4 Stunden bei 80 °C gerührt. Man erhielt ein klares, farbloses Produkt.

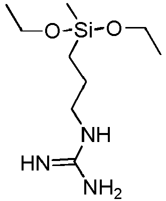

### Beispiel 2

Herstellung von 3-Harnstoffethylaminopropylmethyldiethoxysilan:
In einem 1000-ml-Vierhalskolben mit angeschlossenen KPG-Rührer, Rückflusskühler und Innenthermometer wurden 660,35 g 3-Aminoethylaminopropylmethyldiethoxysilan (Dynasylan 1411, Evonik Industries AG) und 192,19 g Harnstoff (Sigma Aldrich) vorgelegt. Es wurde auf 100 °C erhitzt und 40 h gerührt. Man erhielt ein klares, farbloses Produkt.

### Beispiel 3: Herstellung einer Shampoo-Formulierung, erfindungsgemäß:

Mit dem Silan Beispiel 1 wurde eine Shampoo-Formulierung nach folgender Zusammensetzung hergestellt. Dabei wurde das Guar Quat in Wasser gegeben und quellen gelassen. Diese Mischung wurde unter Rühren bei Raumtemperatur zu der Lösung des Laureth Sulfats, des Betains und des Silans gegeben. Salz wurde hinzugefügt und der pH Wert mit Zitronensäure eingestellt

| **Formulierungsbeispiel 3** | |
|---|---|
| Texapon® NSO, 28%-ig, BASF (INCI: Sodium Laureth Sulfate) | 32,0% |
| TEGO® Betain F 50, 38%-ig, Evonik Industries AG (INCI: Cocamidopropyl Betaine) | 8,0% |
| Jaguar C-162, Solvay Rhodia (INCI: Guar Hydroxypropyl trimonium Chloride) | 0,2% |
| NaCl | 1,0% |
| Wasser, demineralisiert | ad 100,0% |
| Zitronensäure | ad. pH 6,0 ± 0,3 |
| Silan Beispiel 1 | 1,0% |

### Beispiel 4: Herstellung einer Haarspülungs-Formulierung, erfindungsgemäß:

Mit dem Silan Beispiel 1 wurde eine Haarspülungs-Formulierung nach folgender Zusammensetzung hergestellt. Dabei wurden das TEGINACID C, der Fettalkohol und das Silan auf 75 °C aufgeheizt und mit der Wasserphase (CTAC in Wasser) versetzt und mit einem Stabmixer homogenisiert. Unter Rühren wurde abgekühlt und bei <40 °C das Konservierungsmittel zugegeben. Mit Zitronensäure wurde schließlich der pH Wert eingestellt.

| **Formulierung Beispiel 4** | |
|---|---|
| TEGINACID® C, Evonik Industries AG (INCI: Ceteareth-25) | 0,5% |
| TEGO® Alkanol 1618, Evonik Industries AG (INCI: Cetearyl Alcohol) | 5,0% |
| Silan Beispiel 1 | 1,0% |
| VARISOFT® 300, 30%-ig, Evonik Industries AG (INCI: Cetrimonium Chloride (=CTAC)) | 1,0% |
| Neolone PE, The Dow Chemical Company (INCI: Phenoxyethanol; Methylisothiazolinone) | 0,45% |
| Wasser, demineralisiert | ad. 100,0% |
| Zitronensäure | ad. pH 4,5 ± 0,3 |

### Beispiel 5: Herstellung einer Haarspülungs-Formulierung, erfindungsgemäß:

Mit dem Silan Beispiel 2 wurde eine Haarspülungs-Formulierung nach folgender Zusammensetzung hergestellt. Dabei wurden das TEGINACID C, der Fettalkohol und das Silan auf 75 °C aufgeheizt und mit der Wasserphase (CATC in Wasser) versetzt und mit einem Stabmixer homogenisiert. Unter Rühren wurde abgekühlt und bei <40 °C das Konservierungsmittel zugegeben. Mit Zitronensäure wurde schließlich der pH Wert eingestellt.

| **Formulierung Beispiel 5** | |
|---|---|
| TEGINACID® C, Evonik Industries AG (INCI: Ceteareth-25) | 0,5% |
| TEGO® Alkanol 1618, Evonik Industries AG (INCI: Cetearyl Alcohol) | 5,0% |
| Silan Beispiel 2 | 1,0% |
| VARISOFT® 300, 30%-ig, Evonik Industries AG (INCI: Cetrimonium Chloride (=CTAC)) | 1,0% |
| Neolone PE, The Dow Chemical Company (INCI: Phenoxyethanol; Methylisothiazolinone) | 0,45% |
| Wasser, demineralisiert | ad. 100,0% |
| Zitronensäure | ad. pH 4,5 ± 0,3 |

### Beispiel 6: Herstellung einer Shampoo-Formulierung, nicht erfindungsgemäß:

Mit 3-Aminopropyltriethoxysilan (Dynasylan AMEO, Evonik Industries AG) wurde eine Shampoo-Formulierung nach folgender Zusammensetzung hergestellt. Dabei wurde das Guar Quat in Wasser gegeben und quellen gelassen. Diese Mischung wurde unter Rühren bei Raumtemperatur zu der Lösung des Laureth Sulfats, des Betains und des Silans gegeben. Salz wurde hinzugefügt und der pH Wert mit Zitronensäure eingestellt

| **Formulierungsbeispiel 6** | |
|---|---|
| Texapon NSO® 28%-ig, BASF (INCI: Sodium Laureth Sulfate) | 32,0% |
| TEGO® Betain F 50, 38%-ig, Evonik Industries AG (INCI: Cocamidopropyl Betaine) | 8,0% |
| Jaguar C-162, Solvay Rhodia (INCI: Guar Hydroxypropyl trimonium Chloride) | 0,2% |
| NaCl | 1,0% |
| Wasser, demineralisiert | ad 100,0% |
| Zitronensäure | ad. pH 6,0 ± 0,3 |
| 3-Aminopropyltriethoxysilan | 1,0% |

### Beispiel 7: Herstellung einer Haarspülungs-Formulierung, nicht erfindungsgemäß:

Mit 3-Aminopropyltriethoxysilan (Dynasylan AMEO, Evonik Industries AG) wurde eine Haarspülungs-Formulierung nach folgender Zusammensetzung hergestellt. Dabei wurden das TEGINACID C, der Fettalkohol und das Silan auf 75 °C aufgeheizt und mit der Wasserphase (CATC in Wasser) versetzt und mit einem Stabmixer homogenisiert. Unter Rühren wurde abgekühlt und bei <40 °C das Konservierungsmittel zugegeben. Mit Zitronensäure wurde schließlich der pH Wert eingestellt.

| **Formulierung Beispiel 7** | |
|---|---|
| TEGINACID® C, Evonik Industries AG (INCI: Ceteareth-25) | 0,5% |
| TEGO® Alkanol 1618, Evonik Industries AG (INCI: Cetearyl Alcohol) | 5,0% |
| 3-Aminopropyltriethoxysilan | 1,0% |
| VARISOFT® 300, 30%-ig, Evonik Industries (INCI: Cetrimonium Chloride (=CTAC)) | 1,0% |
| Neolone PE, The Dow Chemical Company (INCI: Phenoxyethanol; Methylisothiazolinone) | 0,45% |
| Wasser, demineralisiert | ad. 100,0% |
| Zitronensäure | ad. pH 4,5 ± 0,3 |

### Beispiel 8: Anwendungstests

Für die anwendungstechnische sensorische Beurteilung wurden Haartressen (Fa. Kerling, Deutschland) durch eine Bleichbehandlung standardisiert vorgeschädigt. Dazu wurden friseurübliche Produkte verwendet. Der Testablauf, die verwendeten Basismaterialien sowie die Details der Beurteilungskriterien wurden in DE 103 27 871 beschrieben.

Die Vorbehandlung der Haare erfolgte durch ein Shampoo, welches keine Konditioniermittel enthält.

### Standardisierte Behandlung von vorgeschädigten Haartressen mit konditionierenden Shampoos:

Die, wie oben beschrieben, vorgeschädigten Haartressen wurden wie folgt mit den oben beschriebenen Shampoos behandelt:
Die Haartressen wurden unter fließendem, warmem Wasser (38 °C, 10 °dH) benetzt. Das überschüssige Wasser wurde leicht von Hand ausgedrückt, dann wird das Vorbehandlungs-Shampoo aufgebracht und sanft im Haar eingearbeitet (1 ml/Haartresse (2 g)). Nach einer Verweilzeit von 1 min wurde das Haar für 1 min gespült. Direkt im Anschluss wurde das konditionierende Shampoo aufgebracht und sanft im Haar eingearbeitet (1 ml/Haartresse (2 g)). Nach einer Verweilzeit von 1 min wurde das Haar für 1 min gespült. Zur Bewertung der Eigenschaften der Shampooformulierung wurde im Testablauf keine Nachbehandlung mit einer Spülung durchgeführt.

### Standardisierte Behandlung von vorgeschädigten Haartressen mit konditionierenden Haarspülungs-Formulierungen:

Die, wie oben beschrieben, vorgeschädigten Haartressen wurden wie folgt mit den oben beschriebenen konditionierenden Haarspülungen behandelt:
Die Haartressen wurden unter fließendem, warmem Wasser (38 °C, 10 °dH) benetzt. Das überschüssige Wasser wurde leicht von Hand ausgedrückt, dann wird das Vorbehandlungs-Shampoo aufgebracht und sanft im Haar eingearbeitet (1 ml/Haartresse (2 g)). Nach einer Verweilzeit von 1 min wurde das Haar für 1 min gespült. Direkt im Anschluss wurde die Spülung aufgebracht und sanft im Haar eingearbeitet (1 ml/Haartresse (2 g)). Nach einer Verweilzeit von 1 min wurde das Haar für 1 min gespült.

### Beurteilungskriterien:

Die sensorischen Bewertungen erfolgten nach Noten, die auf einer Skala von 1 bis 5 vergeben wurden, wobei 1 die schlechteste und 5 die beste Bewertung ist. Die einzelnen Testkriterien erhielten jeweils eine eigene Bewertung.

Die Testkriterien sind: Nasskämmbarkeit, Nassgriff, Trockenkämmbarkeit, Trockengriff, Aussehen/Glanz.

### Anwendungstests Shampoo, vergleichend erfindungsgemäß /nicht erfindungsgemäß:

Die anwendungstechnischen Eigenschaften der erfindungsgemäßen Verbindungen beim Einsatz in Shampoos wurden in den oben beschriebenen Formulierungen Beispiel 3 und 6 überprüft.

In der folgenden Tabelle wurden die Ergebnisse der sensorischen Beurteilung der wie oben beschrieben mit der erfindungsgemäßen Formulierung 3, der Vergleichsformulierung 6 und der Kontrollformulierung 0a (Placebo ohne konditionierende Silan-Substanz) behandelten Haarsträhnchen verglichen.

| | **Nasskämm-barkeit** | **Nass-griff** | **Trockenkämmbarkeit** | **Trocken-griff** | **Glanz** |
|---|---|---|---|---|---|
| **Kontrollformulierung 0a** | 2,5 | 2,8 | 3,9 | 4,0 | 3,0 |
| **Erfindungsgemäße Formulierung 3** | 3,2 | 3,7 | 4,3 | 4,3 | 4,0 |
| **Vergleichsformulierung 6 (nicht erfindungsgemäß)** | 3,1 | 3,5 | 4,0 | 4,0 | 3,5 |

Die erfindungsgemäße Formulierung 3 zeigte in der sensorischen Beurteilung gute kosmetische Bewertungen. Die Kontrollformulierung 0a (mit Guar Quat) wurde durch die Zugabe des Silans 1 signifikant verbessert. Dabei wurden die bereits guten Eigenschaften der Vergleichsformulierung 6 vor allem bzgl. Trockenkämmbarkeit, Trockengriff und Glanz durch die erfindungsgemäße Formulierung 3 noch weiter gesteigert.

### Anwendungstests Haarspülung, vergleichend erfindungsgemäß /nicht erfindungsgemäß:

Die anwendungstechnischen Eigenschaften der erfindungsgemäßen Verbindungen beim Einsatz in Haarspülungen wurden in den oben beschriebenen Formulierungen Beispiel 4, 5 und 7 überprüft.

In der folgenden Tabelle wurden die Ergebnisse der sensorischen Beurteilung der wie oben beschrieben mit den erfindungsgemäßen Formulierungen 4 und 5, der Vergleichsformulierung 7 und der Kontrollformulierung 0b (Placebo ohne konditionierende Silan Substanz) behandelten Haarsträhnchen verglichen.

| | **Nasskämm-barkeit** | **Nass-griff** | **Trockenkämmbarkeit** | **Trocken-griff** | **Glanz** |
|---|---|---|---|---|---|
| **Kontrollformulierung 0b** | 3,8 | 3,7 | 3,7 | 4,0 | 3,0 |
| **Erfindungsgemäße Formulierung 4** | 4,3 | 4,2 | 4,2 | 4,5 | 4,0 |
| **Erfindungsgemäße Formulierung 5** | 4,2 | 4,1 | 4,2 | 4,4 | 4,0 |
| **Vergleichsformulierung 7 (nicht erfindungsgemäß)** | 4,1 | 4,0 | 4,1 | 4,2 | 3,5 |

Die erfindungsgemäßen Formulierungen 4 und 5 zeigten in der sensorischen Beurteilung gute kosmetische Bewertungen. Die Kontrollformulierung 0b (mit CTAC) wurde durch die Zugabe der Silane 1 oder 2 signifikant verbessert. Dabei wurden die bereits guten Eigenschaften der Vergleichsformulierung 7 vor allem bzgl. Trockengriff und Glanz durch die erfindungsgemäßen Formulierungen 4 und 5 noch weiter gesteigert.

### Beispiel 9: Weitere Formulierungsbeispiele

**Formulierungsbeispiel 91: Shampoo**

| | |
|---|---|
| TEXAPON® NSO, BASF, 28%-ig (INCI: Sodium Laureth Sulfate) | 32,00% |
| Silan Beispiel 1 | 0,80% |
| Perfume | 0,50% |
| Water | 57,00% |
| TEGO® Betain F 50, Evonik Industries AG, 38%-ig (INCI: Cocamidopropyl Betaine) | 8,00% |
| ANTIL® 171, Evonik Industries AG (INCI: PEG-18 Glyceryl Oleate/Cocoate) | 1,00% |
| NaCl | 0,70% |
| Preservative | q.s. |

**Formulierungsbeispiel 92: Conditioning Shampoo**

| | |
|---|---|
| TEXAPON® NSO, BASF, 28%-ig (INCI: Sodium Laureth Sulfate) | 32,00% |
| Silan Beispiel 1 | 1,00% |
| Perfume | 0,50% |
| Water | 56,90% |
| Jaguar C-162, Solvay Rhodia (INCI: Hydroxypropyl Guar Hydroxypropyltrimonium Chloride) | 0,30% |
| TEGO® Betain F 50, Evonik Industries AG, 38%-ig (INCI: Cocamidopropyl Betaine) | 8,00% |
| NaCl | 1,30% |
| Preservative | q.s. |

**Formulierungsbeispiel 93: Conditioning Shampoo**

| | |
|---|---|
| TEXAPON® NSO, BASF, 28%-ig (INCI: Sodium Laureth Sulfate) | 32,00% |
| ANTIL® 200, Evonik Industries AG (INCI: PEG-200 Hydrogenated Glyceryl Palmate; PEG-7 Glyceryl Cocoate) | 2,00% |
| Silan Beispiel 1 | 1,00% |
| Perfume | 0,25% |
| Water | 57,25% |
| Polymer JR 400, Amerchol (INCI: Polyquaternium-10) | 0,20% |
| TEGO® Betain C 60, Evonik Industries AG, 47%-ig (INCI: Cocamidopropyl Betaine) | 6,00% |
| NaCl | 1,30% |
| Preservative | q.s. |

**Formulierungsbeispiel 94: Conditioning Shampoo**

| | |
|---|---|
| TEXAPON® NSO, BASF, 28%-ig (INCI: Sodium Laureth Sulfate) | 32,00% |
| ANTIL® 200, Evonik Industries AG (INCI: PEG-200 Hydrogenated Glyceryl Palmate; PEG-7 Glyceryl Cocoate) | 2,00% |
| ABIL® Quat 3272, Evonik Industries AG, 50%-ig (INCI: Quaternium-80) | 0,75% |
| Silan Beispiel 2 | 1,00% |
| Perfume | 0,25% |
| Water | 55,50% |
| Polymer JR 400, Amerchol (INCI: Polyquaternium-10) | 0,20% |
| TEGO® Betain F 50, Evonik Industries AG, 38%-ig (INCI: Cocamidopropyl Betaine) | 8,00% |
| NaCl | 0,30% |
| Preservative | q.s. |

**Formulierungsbeispiel 95: Conditioning Shampoo**

| | |
|---|---|
| TEXAPON® NSO, BASF, 28%-ig (INCI: Sodium Laureth Sulfate) | 32,00% |
| VARISOFT® PATC, Evonik Industries AG (INCI: Palmitamidopropyltrimonium Chloride) | 1,50% |
| REWODERM® LI S 80, Evonik Industries AG (INCI: PEG-200 Hydrogenated Glyceryl Palmate; PEG-7 Glyceryl Cocoate) | 2,00% |
| Silan Beispiel 1 | 0,50% |
| Perfume | 0,25% |
| Water | 55,15% |
| TEGO® Cosmo C 100, Evonik Industries AG (INCI: Creatine) | 1,00% |
| Jaguar C-162, Solvay Rhodia (INCI: Hydroxypropyl Guar Hydroxypropyltrimonium Chloride) | 0,20% |
| TEGO® Betain F 50, Evonik Industries AG, 38%-ig (INCI: Cocamidopropyl Betaine) | 7,00% |
| NaCl | 0,40% |
| Preservative | q.s. |

**Formulierungsbeispiel 96: Pearlized Shampoo**

| | |
|---|---|
| TEXAPON® NSO, BASF, 28%-ig (INCI: Sodium Laureth Sulfate) | 32,00% |
| Silan Beispiel 2 | 1,50% |
| Perfume | 0,25% |
| Water | 54,25% |
| TEGO® Betain F 50, Evonik Industries AG, 38%-ig (INCI: Cocamidopropyl Betaine) | 8,00% |
| TEGO® Pearl N 300, Evonik Industries AG (INCI: Glycol Distearate; Laureth-4; Cocamidopropyl Betaine) | 2,00% |
| ANTIL® 171, Evonik Industries AG (INCI: PEG-18 Glyceryl Oleate/Cocoate) | 1,50% |
| NaCl | 0,50% |
| Preservative | q.s. |

**Formulierungsbeispiel 97: 2 in 1 Shampoo**

| | |
|---|---|
| TEXAPON® NSO, BASF, 28%-ig (INCI: Sodium Laureth Sulfate) | 32,00% |
| VARISOFT® PATC, Evonik Industries AG (INCI: Palmitamidopropyltrimonium Chloride) | 1,30% |
| REWODERM® LI S 80, Evonik Industries AG (INCI: PEG-200 Hydrogenated Glyceryl Palmate; PEG-7 Glyceryl Cocoate) | 2,00% |
| Silan Beispiel 1 | 0,50% |
| Silan Beispiel 2 | 0,50% |
| Perfume | 0,25% |
| Water | 54,05% |
| TEGO® Cosmo C 100, Evonik Industries AG (INCI: Creatine) | 0,50% |
| Jaguar C-162, Solvay Rhodia (INCI: Hydroxypropyl Guar Hydroxypropyltrimonium Chloride) | 0,20% |
| TEGO® Betain F 50, Evonik Industries AG, 38%-ig (INCI: Cocamidopropyl Betaine) | 8,00% |
| NaCl | 0,70% |
| Preservative | q.s. |

**Formulierungsbeispiel 98: Rinse-Off Conditioner**

| | |
|---|---|
| Water | 90,50% |
| VARISOFT® BT 85, Evonik Industries AG (INCI: Behentrimonium Chloride) | 3,00% |
| Silan Beispiel 2 | 1,50% |
| TEGO® Alkanol 1618, Evonik Industries AG (INCI: Cetearyl Alcohol) | 5,00% |
| Preservative, Perfume | q.s. |

**Formulierungsbeispiel 99: Rinse-Off Conditioner**

| | |
|---|---|
| Water | 90,10% |
| VARISOFT® EQ 65, Evonik Industries AG (INCI: Distearyl Dimonium Chloride; Cetearyl Alcohol) | 2,00% |
| VARISOFT® BT 85, Evonik Industries AG (INCI: Behentrimonium Chloride) | 2,00% |
| Silan Beispiel 1 | 0,90% |
| TEGO® Alkanol 1618, Evonik Industries AG (INCI: Cetearyl Alcohol) | 5,00% |
| Preservative, Perfume | q.s. |

**Formulierungsbeispiel 910: Rinse-Off Conditioner**

| | |
|---|---|
| Water | 89,70% |
| VARISOFT® EQ 65, Evonik Industries AG (INCI: Distearyl Dimonium Chloride; Cetearyl Alcohol) | 2,00% |
| VARISOFT® BT 85, Evonik Industries AG (INCI: Behentrimonium Chloride) | 2,00% |
| ABIL® Quat 3272, Evonik Industries AG, 50%-ig (INCI: Quaternium-80) | 0,50% |
| Silan Beispiel 1 | 0,80% |
| TEGO® Alkanol 1618, Evonik Industries AG (INCI: Cetearyl Alcohol) | 4,50% |
| Dodecanol, Aldrich | 0.50% |
| Preservative, Perfume | q.s. |

**Formulierungsbeispiel 911: Rinse-Off Conditioner**

| | |
|---|---|
| TEGINACID® C, Evonik Industries AG (INCI: Ceteareth-25) | 0,40% |
| TEGO® Alkanol 16, Evonik Industries AG (INCI: Cetyl Alcohol) | 2,00% |
| TEGO® Amid S 18, Evonik Industries AG (INCI: Stearamidopropyl Dimethylamine) | 1,00% |
| Silan Beispiel 2 | 1,50% |
| Propylene Glycol | 2,00% |
| Citric Acid Monohydrate | 0,30% |
| Water | 92,80% |
| Preservative, Perfume | q.s. |

**Formulierungsbeispiel 912: Rinse-Off Conditioner**

| | |
|---|---|
| TEGINACID® C, Evonik Industries AG (INCI: Ceteareth-25) | 0,50% |
| TEGO® Alkanol 1618, Evonik Industries AG (INCI: Cetearyl Alcohol) | 5,00% |
| Dodecanol, Aldrich | 1,00% |
| TEGOSOFT® DEC, Evonik Industries AG (INCI: Diethylhexyl Carbonate) | 1,00% |
| Silan Beispiel 1 | 1,50% |
| Water | 88,20% |
| TEGO® Cosmo C 100, Evonik Industries AG (INCI: Creatine) | 0,50% |
| Propylene Glycol | 2,00% |
| Citric Acid Monohydrate | 0,30% |
| Preservative, Perfume | q.s. |

**Formulierungsbeispiel 913: Leave-In Conditioner Spray**

| | |
|---|---|
| Lactic Acid, 80% | 0,40% |
| Water | 95,50% |
| TEGO® Amid S 18, Evonik Industries AG (INCI: Stearamidopropyl Dimethylamine) | 1,20% |
| TEGIN® G 1100 Pellets, Evonik Industries AG (INCI: Glycol Distearate) | 0,90% |
| TEGO® Care PS, Evonik Industries AG (INCI: Methyl Glucose Sesquistearate) | 1,20% |
| TEGOSOFT® DEC, Evonik Industries AG (INCI: Diethylhexyl Carbonate) | 0,30% |
| Silan Beispiel 1 | 0,50% |
| Preservative, Perfume | q.s. |

**Formulierungsbeispiel 914: Leave-In Conditioner Spray**

| | |
|---|---|
| TAGAT® CH-40, Evonik Industries AG (INCI: PEG-40 Hydrogenated Castor Oil) | 2,50% |
| Ceramide VI, Evonik Industries AG (INCI: Ceramide 6 II) | 0,05% |
| Perfume | 0,20% |
| Water | 90,45% |
| Silan Beispiel 2 | 0,50% |
| LACTIL®, Evonik Industries AG (INCI: Sodium Lactate; Sodium PCA; Glycine; Fructose; Urea; Niacinamide; Inositol; Sodium Benzoate; Lactic Acid) | 2,00% |
| TEGO® Betain F 50, Evonik Industries AG, 38%-ig (INCI: Cocamidopropyl Betaine) | 2,30% |
| Citric Acid (10% in water) | 2,00% |

**Formulierungsbeispiel 915: Creamy Shaving Foam**

| | |
|---|---|
| Water | 50,40% |
| Coconut Fatty Acid | 1,40% |
| Monoethanolamine | 1,30% |
| Myristic Acid | 3,50% |
| TEGOSOFT® LSE 65 K Evonik Industries AG (INCI: Sucrose Cocoate) | 2,00% |
| TEGO® Betain 810 Evonik Industries AG (INCI: Capryl/Capramidopropyl Betaine) | 7,60% |
| Glycerin | 5,00% |
| Silan Beispiel 1 | 1,30% |
| Perfume | 0,30% |
| Water | 26,50% |
| TEGOCEL® HPM 50, Evonik Industries AG (INCI: Hydroxypropyl Methylcellulose) | 0,70% |

**Formulierungsbeispiel 916: Body Wash**

| | |
|---|---|
| TEXAPON® NSO, BASF, 28%-ig (INCI: Sodium Laureth Sulfate) | 30,00% |
| TEGOSOFT® PC 31, Evonik Industries AG (INCI: Polyglyceryl-3 Caprate) | 0,50% |
| Silan Beispiel 2 | 0,30% |
| Perfume | 0,30% |
| Water | 54,60% |
| TEGOCEL® HPM 4000, Evonik Industries AG (INCI: Hydroxypropyl Methylcellulose) | 0,30% |
| REWOTERIC® AM C, Evonik Industries AG, 32%-ig (INCI: Sodium Cocoamphoacetate) | 10,00% |
| Citric Acid Monohydrate | 0,50% |
| REWODERM® LI S 80, Evonik Industries AG (INCI: PEG-200 Hydrogenated Glyceryl Palmate; PEG-7 Glyceryl Cocoate) | 2,00% |
| TEGO® Pearl N 300, Evonik Industries AG (INCI: Glycol Distearate; Laureth-4; Cocamidopropyl Betaine) | 1,50% |

**Formulierungsbeispiel 917: Mild Shower Bath**

| | |
|---|---|
| TEXAPON® NSO, BASF, 28%-ig (INCI: Sodium Laureth Sulfate) | 27,00% |
| REWOPOL® SB FA 30, Evonik Industries AG, 40%-ig (INCI: Disodium Laureth Sulfosuccinate) | 12,00% |
| TEGOSOFT® LSE 65 K SOFT, Evonik Industries AG (INCI: Sucrose Cocoate) | 2,00% |
| Water | 39,50% |
| REWOTERIC® AM C, Evonik Industries AG, 32%-ig (INCI: Sodium Cocoamphoacetate) | 13,00% |
| Silan Beispiel 1 | 0,50% |
| Citric Acid (30% in water) | 2,50% |
| ANTIL® 171, Evonik Industries AG (INCI: PEG-18 Glyceryl Oleate/Cocoate) | 1,50% |
| TEGO® Pearl N 300, Evonik Industries AG (INCI: Glycol Distearate; Laureth-4; Cocamidopropyl Betaine) | 2,00% |

**Formulierungsbeispiel 918: Strong Hold Styling Gel**

| | |
|---|---|
| TEGO® Carbomer 141, Evonik Industries AG (INCI: Carbomer) | 1,20% |
| Water | 67,20% |
| NaOH, 25% | 2,50% |
| PVP/VA W-735, ISP (INCI: PVP/VA Copolymer) | 16,00% |
| Silan Beispiel 1 | 0,50% |
| Alcohol Denat. | 10,00% |
| TAGAT® O 2 V, Evonik Industries AG (INCI: PEG-20 Glyceryl Oleate) | 2,00% |
| Perfume | 0,30% |
| ABIL® B 88183, Evonik Industries AG (INCI: PEG/PPG-20/6 Dimethicone) | 0,30% |
| Preservative | q.s. |

**Formulierungsbeispiel 919: Körperpflegemittel**

| | |
|---|---|
| TEXAPON® NSO, BASF, 28%-ig (INCI: Sodium Laureth Sulfate) | 30,00% |
| TEGOSOFT® PC 31, Evonik Industries AG (INCI: Polyglyceryl-3 Caprate) | 0,50% |
| Silan Beispiel 2 | 0,50% |
| Perfume | 0,30% |
| Water | 53,70% |
| TEGOCEL® HPM 4000, Evonik Industries AG (INCI: Hydroxypropyl Methylcellulose) | 0,50% |
| REWOTERIC® AM C, Evonik Industries AG, 32%-ig (INCI: Sodium Cocoamphoacetate) | 10,00% |
| Citric Acid Monohydrate | 0,50% |
| REWODERM® LI S 80, Evonik Industries AG (INCI: PEG-200 Hydrogenated Glyceryl Palmate; PEG-7 Glyceryl Cocoate) | 2,00% |
| TEGO® Pearl N 300, Evonik Industries AG (INCI: Glycol Distearate; Laureth-4; Cocamidopropyl Betaine) | 2,00% |

**Formulierungsbeispiel 920: Schaumiges Körperpflegemittel**

| | |
|---|---|
| TEXAPON® NSO, BASF, 28%-ig (INCI: Sodium Laureth Sulfate) | 14,30% |
| Perfume | 0,30% |
| Silan Beispiel 1 | 1,00% |
| REWOTERIC® AM C, Evonik Industries AG, 32%-ig (INCI: Sodium Cocoamphoacetate) | 8,00% |
| Water | 74,40% |
| Jaguar C-162, Solvay Rhodia (INCI: Hydroxypropyl Guar Hydroxypropyltrimonium Chloride) | 0,20% |
| Polyquaternium-7 | 0,30% |
| LACTIL®, Evonik Industries AG (INCI: Sodium Lactate; Sodium PCA; Glycine; Fructose; Urea; Niacinamide; Inositol; Sodium Benzoate; Lactic Acid) | 1,00% |
| Citric Acid Monohydrate | 0,50% |

**Formulierungsbeispiel 921: Mild Foam Bath**

| | |
|---|---|
| TEXAPON® NSO, BASF, 28%-ig (INCI: Sodium Laureth Sulfate) | 27,00% |
| REWOPOL® SB FA 30, Evonik Industries AG, 40%-ig (INCI: Disodium Laureth Sulfosuccinate) | 12,00% |
| TEGOSOFT® LSE 65 K SOFT, Evonik Industries AG (INCI: Sucrose Cocoate) | 2,00% |
| Water | 39,00% |
| REWOTERIC® AM C, Evonik Industries AG, 32%-ig (INCI: Sodium Cocoamphoacetate) | 13,00% |
| Silan Beispiel 2 | 0,50% |
| Citric Acid (30% in water) | 3,00% |
| ANTIL® 171, Evonik Industries AG (INCI: PEG-18 Glyceryl Oleate/Cocoate) | 1,50% |
| TEGO® Pearl N 300, Evonik Industries AG (INCI: Glycol Distearate; Laureth-4; Cocamidopropyl Betaine) | 2,00% |

**Formulierungsbeispiel 922: Schaumiges Körperpflegemittel**

| | |
|---|---|
| TEGOCEL® HPM 50, Evonik Industries AG (INCI: Hydroxypropyl Methylcellulose) | 0,50% |
| Water | 80,50% |
| Perfume | 0,20% |
| Silan Beispiel 1 | 0,60% |
| TEGOSOFT® GC, Evonik Industries AG (INCI: PEG-7 Glyceryl Cocoate) | 1,30% |
| TEGO® Betain 810, Evonik Industries AG (INCI: Capryl/Capramidopropyl Betaine) | 16,90% |
| Preservative | q.s. |

**Formulierungsbeispiel 923: Rinse-Off Conditioner**

| | |
|---|---|
| Water | 89,30% |
| VARISOFT® EQ 65, Evonik Industries AG (INCI: Distearyl Dimonium Chloride; Cetearyl Alcohol) | 2,00% |
| VARISOFT® BT 85, Evonik Industries AG (INCI: Behentrimonium Chloride) | 2,00% |
| ABIL® OSW 5, Evonik Industries AG (INCI: Cyclopentasiloxane; Dimethiconol) | 1,00% |
| Silan Beispiel 1 | 0,70% |
| TEGO® Alkanol 1618, Evonik Industries AG (INCI: Cetearyl Alcohol) | 5,00% |
| Preservative, Perfume | q.s. |

**Formulierungsbeispiel 924: Rinse-Off Conditioner**

| | |
|---|---|
| Water | 89,20% |
| VARISOFT® EQ 65, Evonik Industries AG (INCI: Distearyl Dimonium Chloride; Cetearyl Alcohol) | 2,00% |
| VARISOFT® BT 85, Evonik Industries AG (INCI: Behentrimonium Chloride) | 1,00% |
| ABIL® Soft AF 100, Evonik Industries AG (INCI: Methoxy PEG/PPG-7/3 Aminopropyl Dimethicone) | 1,00% |
| Silan Beispiel 1 | 1,80% |
| TEGO® Alkanol 1618, Evonik Industries AG (INCI: Cetearyl Alcohol) | 5,00% |
| Preservative, Perfume | q.s. |

**Formulierungsbeispiel 925: Rinse-Off Conditioner**

| | |
|---|---|
| Water | 89,20% |
| VARISOFT® EQ 65, Evonik Industries AG (INCI: Distearyl Dimonium Chloride; Cetearyl Alcohol) | 2,00% |
| VARISOFT® BT 85, Evonik Industries AG (INCI: Behentrimonium Chloride) | 2,00% |
| SF 1708, Momentive (INCI: Amodimethicone) | 1,00% |
| Silan Beispiel 1 | 0,80% |
| TEGO® Alkanol 1618, Evonik Industries AG (INCI: Cetearyl Alcohol) | 5,00% |
| Preservative, Perfume | q.s. |

**Formulierungsbeispiel 926: Conditioning Shampoo**

| | |
|---|---|
| TEXAPON® NSO, BASF, 28%-ig (INCI: Sodium Laureth Sulfate) | 27,00% |
| Plantacare 818 UP, BASF, 51,4%-ig (INCI: Coco Glucoside) | 5,00% |
| Silan Beispiel 1 | 1,50% |
| Perfume | 0,25% |
| Water | 56,45% |
| TEGO® Cosmo C 100, Evonik Industries AG (INCI: Creatine) | 1,00% |
| Jaguar C-162, Solvay Rhodia (INCI: Hydroxypropyl Guar Hydroxypropyltrimonium Chloride) | 0,30% |
| TEGO® Betain F 50, Evonik Industries AG, 38%-ig (INCI: Cocamidopropyl Betaine) | 8,00% |
| NaCl | 0,50% |
| Preservative | q.s. |

**Formulierungsbeispiel 927: Conditioning Shampoo**

| | |
|---|---|
| Plantacare 818 UP, BASF, 51,4%-ig (INCI: Coco-Glucoside) | 18,00% |
| Silan Beispiel 2 | 0,50% |
| Perfume | 0,25% |
| Water | 72,55% |
| Jaguar C-162, Solvay Rhodia (INCI: Hydroxypropyl Guar Hydroxypropyltrimonium Chloride) | 0,20% |
| TEGO® Betain F 50, Evonik Industries AG, 38%-ig (INCI: Cocamidopropyl Betaine) | 8,00% |
| NaCl | 0,50% |
| Preservative | q.s. |

**Formulierungsbeispiel 928: Pearlized Shampoo**

| | |
|---|---|
| TEXAPON® NSO, BASF, 28%-ig (INCI: Sodium Laureth Sulfate) | 32,00% |
| Silan Beispiel 2 | 0,75% |
| Perfume | 0,20% |
| Water | 56,05% |
| TEGO® Betain F 50, Evonik Industries AG, 38%-ig (INCI: Cocamidopropyl Betaine) | 8,00% |
| TEGIN® D 1102, Evonik Industries AG (INCI: PEG-3 Distearate) | 1,00% |
| ANTIL® 171, Evonik Industries AG (INCI: PEG-18 Glyceryl Oleate/Cocoate) | 1,50% |
| NaCl | 0,50% |
| Preservative | q.s. |

**Formulierungsbeispiel 929: "Two in One" Shampoo**

| | | |
|---|---|---|
| A | TEXAPON® NSO, BASF, 28%-ig, (INCI: Sodium Laureth Sulfate) | 28,00% |
| | Perfume | 0,50% |
| | Stepanate® SCS, Stepan, (INCI: Sodium Cumenesulfonate) | 1,00% |
| | Water | 18,25% |
| | TEGO® Betain F 50, Evonik Industries AG, 38%-ig, (INCI: Cocamidopropyl Betaine) | 8,00% |
| B | TEXAPON® NSO, BASF, 28%-ig, (INCI: Sodium Laureth Sulfate) | 14,00% |
| | Stepanate® SCS, Stepan, (INCI: Sodium Cumenesulfonate) | 1,00% |
| | Water | 6,00% |
| | REWOMID® C 212, Evonik Industries AG, (INCI: Cocamide MEA) | 1,50% |
| | TEGIN® G 1100 Pellets, Evonik Industries AG, (INCI: Glycol Distearate) | 1,50% |
| C | TEXAPON® NSO, BASF, 28%-ig, (INCI: Sodium Laureth Sulfate) | 15,00% |
| | TEGO® Alkanol 16, Evonik Industries AG, (INCI: Cetyl Alcohol) | 0,50% |
| | Silan Beispiel 2 | 1,50% |
| | Dimethicone (10000 mPa·s) | 1,50% |
| | Stepanate® SCS, Stepan, (INCI: Sodium Cumenesulfonate) | 1,00% |
| | Keltrol®, CP Telco, (INCI: Xanthan Gum) | 0,75% |
| | Preservative | q.s. |

**Formulierungsbeispiel 930: Conditioning Anti-Schuppen Shampoo**

| | | |
|---|---|---|
| A | TEGIN® G 1100 Pellets, Evonik Industries AG, (INCI: Glycol Distearate) | 3,00% |
| | TEXAPON® NSO, BASF, 28%-ig, (INCI: Sodium Laureth Sulfate) | 40,00% |
| B | Perfume | 0,30% |
| | Zinc-Pyrion NF, WeylChem, 48%-ig, (INCI: Zinc Pyrithione) | 2,00% |
| | Silan Beispiel 2 | 2,00% |
| C | Water | 36,00% |
| | TEGO® Carbomer 341 ER, Evonik Industries AG, (INCI: Acrylates/C10-30 Alkyl Acrylate Crosspolymer) | 0,20% |
| | NaOH, 25%-ig | 0,30% |
| D | REWOTERIC® AM B U 185, Evonik Industries AG, 30%-ig, (INCI: Undecylenamidopropyl Betaine) | 12,50% |
| | ANTIL® SPA 80, Evonik Industries AG, (INCI: Isostearamide MIPA; Glyceryl Laurate) | 3,70% |
| E | Preservative | q.s. |

**Formulierungsbeispiel 931: Shampoo**

| | |
|---|---|
| TEXAPON® NSO, BASF, 28%-ig (INCI: Sodium Laureth Sulfate) | 32,00% |
| Silan Beispiel 2 | 0,55% |
| Perfume | 0,20% |
| Water | 53,35% |
| TEGO® Betain AB 1214, Evonik Industries AG, 31%-ig (INCI: Coco-Betaine) | 8,00% |
| Texapon® K12G, BASF, (INCI: Sodium Lauryl Sulfate) | 2,50% |
| TEGIN® G 1100 Pellets, Evonik Industries AG (INCI: Glycol Distearate) | 1,00% |
| Jaguar C-162, Solvay Rhodia (INCI: Hydroxypropyl Guar Hydroxypropyltrimonium Chloride) | 0,30% |
| TEGO® Carbomer 841 SER, Evonik Industries AG, (INCI: Acrylates/C10-30 Alkyl Acrylate Crosspolymer) | 0,40% |
| Alcohol | 0,30% |
| Lactic Acid | 0.50% |
| NaCl | 0,90% |
| Preservative | q.s. |

**Formulierungsbeispiel 932: Haarfärbemittel**

| | |
|---|---|
| Water | 57,40% |
| TEGO® Alkanol 1618, Evonik Industries AG, (INCI: Cetearyl Alcohol) | 12,00% |
| Eutanol® G, BASF, (INCI: Octyldodecanol) | 3,00% |
| REWOMID® C 212, Evonik Industries AG, (INCI: Cocamide MEA) | 1,50% |
| Super Hartolan® B, Croda, (INCI: Lanolin Alcohol) | 3,00% |
| Avocadoöl, Henry Lamotte, (INCI: Persea Gratissima Oil) | 1,50% |
| Pristerene® 4960, Uniquema, (INCI: Stearic Acid) | 6,00% |
| EDTA BD, BASF, (INCI: Disodium EDTA) | 0,10% |
| Texapon® K12G, BASF, (INCI: Sodium Lauryl Sulfate) | 0,50% |
| Propylenglycol | 5,00% |
| Timica Silver Sparkle, BASF, (INCI: MICA; Titanium Dioxide) | 1,00% |
| Ammoniaklösung, 25%ig | 6,00% |
| 2,5-Diaminotoluolsulfat, (INCI: Toluene-2,5-Diamine) | 1,40% |
| Rodol® RS, Jos. H. Lowenstein & Sons, (INCI: Resorcinol) | 0,30% |
| HC Blue A42, (INCI: 2,4-Diaminophenoxyethanol di HCl) | 0,10% |
| Natriumsulfit | 0,50% |
| Perfume | 0,20% |
| Silan Beispiel 1 | 0,50% |

**Formulierungsbeispiel 933: Haarfärbemittel**

| | |
|---|---|
| Water | 64,00% |
| TEGO® Alkanol 1618, Evonik Industries AG, (INCI: Cetearyl Alcohol) | 12,00% |
| Super Hartolan® B, Croda, (INCI: Lanolin Alcohol) | 2,50% |
| Meadowfoam® Seed Oil, Fanning, (INCI: Limnanthes Alba) | 1,00% |
| Pristerene® 4960, Uniquema, (INCI: Stearic Acid) | 5,50% |
| EDTA BD, BASF, (INCI: Disodium EDTA) | 0,10% |
| Glycerin | 5,00% |
| Texapon® N 70, BASF, (INCI: Sodium Laureth Sulfate) | 2,00% |
| Monoethanolamin | 4,00% |
| 2,5-Diaminotoluolsulfat, (INCI: Toluene-2,5-Diamine) | 0,90% |
| Rodol® RS, Jos. H. Lowenstein & Sons, (INCI: Resorcinol) | 0,20% |
| Jarocol® 4A3MP, Vivimed Labs, (INCI: 4-Amino-M-Cresol) | 0,60% |
| Rodol® PAOC, Jos. H. Lowenstein & Sons, (INCI: 4-Amino-2-Hydroxytoluene) | 0,50% |
| Uantox® EBATE, Universal Preserv-A-Chem, (INCI: Erythorbic Acid) | 0,50% |
| Perfume | 0,20% |
| Silan Beispiel 2 | 1,00% |

**Formulierungsbeispiel 934: Haarfärbemittel**

| | |
|---|---|
| Water demineralized | 67,50% |
| TEGO® Alkanol 1618, Evonik Industries AG, (INCI: Cetearyl Alcohol) | 10,00% |
| Eutanol® G, BASF, (INCI: Octyldodecanol) | 1,00% |
| REWOMID® C 212, Evonik Industries AG, (INCI: Cocamide MEA) | 2,00% |
| TEGIN® VS, Evonik Industries AG, (INCI: Glyceryl Stearate SE) | 5,00% |
| Fitoderm®, Hispano Quimica S. A., (INCI: Squalane) | 1,00% |
| Coenzyme Q 10 | 0,10% |
| EDTA BD, BASF, (INCI: Disodium EDTA) | 0,10% |
| Texapon® K12G, BASF, (INCI: Sodium Lauryl Sulfate) | 0,10% |
| Propylenglycol | 5,00% |
| Ammoniaklösung, 25%ig | 3,00% |
| Rodol® ERN, Jos. H. Lowenstein & Sons, (INCI: 1-Naphthol) | 0,30% |
| Imexine® OAG, Chimex, (INCI: 2-Methyl-5-Hydroxyethylaminophenol) | 1,00% |
| Colorex® WP5, Teluca, (INCI: 1-Hydroxyethyl 4,5-Diamino Pyrazole Sulfate) | 2,60% |
| Ascorbinsäure | 0,30% |
| Perfume | 0,30% |
| Silan Beispiel 1 (erfindungsgemäß) | 0,70% |

## Patentansprüche

1. Verwendung eines Silans der allgemeinen Formel 1 oder einer Haarpflegeformulierung enthaltend
mindestens ein Silan der allgemeinen Formel 1 enthaltend
zwei oder drei Alkoxygruppen,
einen Rest enthaltend mindestens eine Guanidinogruppe,
mit der Maßgabe, dass eine Alkylgruppe enthalten ist, wenn das Silan zwei Alkoxygruppen enthält,
**dadurch gekennzeichnet, dass** das Silan ausgewählt ist aus solchen der allgemeinen Formel 1
R¹ₙXSi(OR²)₃₋ₙ (Formel 1)
mit
n= 0 oder 1, insbesondere 1
R¹=CH₃,
R² = CH₃ oder CH₂CH₃, insbesondere CH₂CH₃,
X = R³Y,
R³ = lineare oder verzweigte, gesättigte oder ungesättigte, divalente Kohlenwasserstoffgruppe mit 1 bis 20, bevorzugt 1 bis 6 Kohlenstoffatomen und insbesondere eine -(CH₂)₃-Gruppe,
Y = organischer Rest umfassend mindestens eine Guanidinogruppe und zusätzlich mindestens eine weitere Aminogruppe, insbesondere eine primäre oder sekundäre Aminogruppe
zur Erhöhung der Grifffestigkeit und/oder
zur Verbesserung der Nasskämmbarkeit und/oder
zur Erhöhung des Glanzes von Haaren.

2. Verwendung gemäß Anspruch 1, **dadurch gekennzeichnet, dass** das Silan genau eine Alkylgruppe und genau zwei Alkoxygruppen enthält.

3. Verwendung gemäß mindestens einem der vorherigen Ansprüche, **dadurch gekennzeichnet, dass**
die Alkoxygruppen im Silan ausgewählt sind aus Methoxy-,
die Alkylgruppe im Silan ausgewählt ist aus Methyl-,
wobei bevorzugt genau eine Alkylgruppe und genau zwei Alkoxygruppen im Silan enthalten sind.

4. Verwendung gemäß mindestens einem der vorherigen Ansprüche, **dadurch gekennzeichnet, dass** das Silan in der Haarpflegeformulierung in einer Menge von 0,1 Gew.-% bis 20 Gew.-%, bevorzugt von 0,3 Gew.-% bis 10 Gew.-%, besonders bevorzugt von 0,5 Gew.-% bis 5 Gew.-%, enthalten ist.

5. Verwendung gemäß mindestens einem der vorherigen Ansprüche, **dadurch gekennzeichnet, dass** neben den Silanen zusätzlich mindestens ein Disilan der allgemeinen Formel 2
R¹(OR²)₂SiR³ZR³Si(OR²)₂R¹ (Formel 2),
wobei
Z= zweibindiger organischer Rest umfassend mindestens eine Harnstoffgruppe oder mindestens eine Guanidinogruppe und optional zusätzlich mindestens eine weitere Aminogruppe, insbesondere eine primäre oder sekundäre Aminogruppe, bevorzugt ausgewählt aus der Gruppe der Reste der Formeln h-k 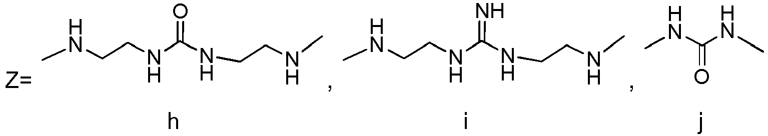 und 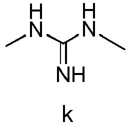 und R¹, R² und R³ wie oben für die allgemeine Formel 1 definiert,
in der Haarpflegeformulierung enthalten ist.

## Claims

1. Use of a silane of the general formula 1 or of a hair care formulation comprising
at least one silane of the general formula 1 containing
two or three alkoxy groups,
a radical containing at least one guanidino group, with the proviso that an alkyl group is present if the silane contains two alkoxy groups,
**characterized in that** the silane is selected from those of the
general formula 1
R¹ₙXSi(OR²)₃₋ₙ (formula 1)
where
n= 0 or 1, in particular 1
R¹ = CH₃,
R² = CH₃ or CH₂CH₃, in particular CH₂CH₃,
X= R³Y,
R³ =linear or branched, saturated or unsaturated, divalent hydrocarbon group with 1 to 20, preferably 1 to 6, carbon atoms and in particular a -(CH₂)₃ group,
Y = organic radical comprising at least one guanidino group and additionally at least one further amino group, in particular a primary or secondary amino group
for increasing the firmness to the touch and/or
for improving the wet combability and/or
for increasing the shine of hair.

2. Use according to Claim 1, **characterized in that** the silane
contains precisely one alkyl group and precisely two alkoxy groups.

3. Use according to at least one of the preceding claims, **characterized in that**
the alkoxy groups in the silane are selected from methoxy,
the alkyl group in the silane is selected from methyl, where preferably precisely one alkyl group and precisely two alkoxy groups are present in the silane.

4. Use according to at least one of the preceding claims, **characterized in that** the silane is present in the hair care formulation in an amount of from 0.1% by weight to 20% by weight, preferably from 0.3% by weight to 10% by weight, particularly preferably from 0.5% by weight to 5% by weight.

5. Use according to at least one of the preceding claims, **characterized in that**, besides the silanes, additionally at least one disilane of the general formula 2
R¹(OR²)₂SiR³ZR³Si(OR²)₂R¹ (formula 2),
where
Z = divalent organic radical comprising at least one urea group or at least one guanidino group and optionally additionally at least one further amino group, in particular a primary or secondary amino group, preferably selected from the group of the radicals of the formulae h-k Z = 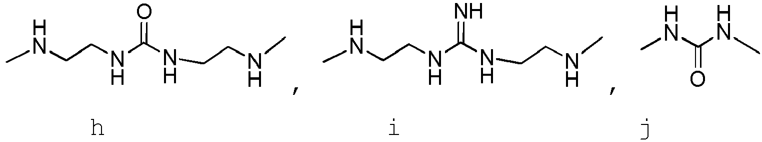 and 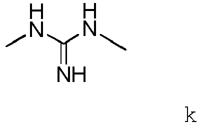 and R¹, R² and R³ are as defined above for the general formula 1,
is present in the hair care formulation.

## Revendications

1. Utilisation d'un silane de formule générale 1 ou d'une formulation de soin des cheveux contenant :
au moins un silane de formule générale 1 contenant :
deux ou trois groupes alcoxy,
un radical contenant au moins un groupe guanidino, à condition qu'un groupe alkyle soit contenu lorsque le silane contient deux groupes alcoxy,
**caractérisée en ce que** le silane est choisi parmi ceux de formule générale 1 :
R¹ₙXSi(OR²)₃₋ₙ (formule 1)
dans laquelle
n = 0 ou 1, notamment 1,
R¹ = CH₃,
R² = CH₃ ou CH₂CH₃, notamment CH₂CH₃,
X = R³Y,
R³ = un groupe hydrocarboné bivalent linéaire ou ramifié, saturé ou insaturé, de 1 à 20, de préférence 1 à 6 atomes de carbone, et notamment un groupe -(CH₂)₃,
Y = un radical organique comprenant au moins un groupe guanidino, et en outre au moins un groupe amino supplémentaire, notamment un groupe amino primaire ou secondaire,
pour l'augmentation de la résistance à la manipulation et/ou
pour l'amélioration de l'aptitude au peignage à l'état humide et/ou
pour l'augmentation de la brillance de cheveux.

2. Utilisation selon la revendication 1, **caractérisée en ce que** le silane contient exactement un groupe alkyle et exactement deux groupes alcoxy.

3. Utilisation selon au moins l'une quelconque des revendications précédentes, **caractérisée en ce que** les groupes alcoxy dans le silane sont choisis parmi méthoxy,
le groupe alkyle dans le silane est choisi parmi méthyle, de préférence exactement un groupe alkyle et exactement deux groupes alcoxy étant contenus dans le silane.

4. Utilisation selon au moins l'une quelconque des revendications précédentes, **caractérisée en ce que** le silane est contenu dans la formulation de soin des cheveux en une quantité de 0,1 % en poids à 20 % en poids, de préférence de 0,3 % en poids à 10 % en poids, de manière particulièrement préférée de 0,5 % en poids à 5 % en poids.

5. Utilisation selon au moins l'une quelconque des revendications précédentes, **caractérisée en ce qu'**en plus des silanes, également au moins un disilane de formule générale 2 :
R¹(OR²)₂SiR³ZR³Si(OR²)₂R¹ (formule 2)
dans laquelle
Z = un radical organique bivalent comprenant au moins un groupe urée ou au moins un groupe guanidino et éventuellement en outre au moins un groupe amino supplémentaire, notamment un groupe amino primaire ou secondaire, de préférence choisi dans le groupe des radicaux des formules h à k : 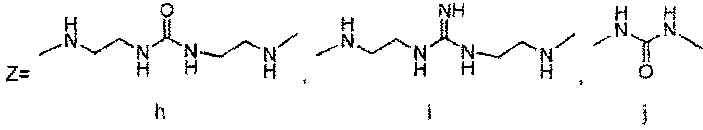 et 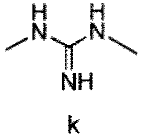 et R¹, R² et R³ sont tels que définis précédemment pour la formule générale 1,
est contenu dans la formulation de soin des cheveux.
